# EUROPEAN PATENT SPECIFICATION

(11) **EP 0 357 404 B1**
(45) Date of publication and mention of the grant of the patent: **12.10.1994**
(21) Application number: 89308780.9
(22) Date of filing: 30.08.1989
(51) Int. Cl.: C07D 249/08, A01N 43/653

(54) **Process for the preparation of cyclopentane derivatives**
Verfahren zur Herstellung von Cyclopentanderivaten
Procédé de préparation de dérivés de cyclopentane

(30) Priority: 31.08.1988 GB 8820607
(43) Date of publication of application: 07.03.1990
(73) Proprietor: KUREHA KAGAKU KOGYO KABUSHIKI KAISHA, Chuo-ku Tokyo 103 (JP)
(72) Inventor: Briner, Paul Howard, Canterbury, Kent CT4 8EY (GB)
(74) Representative: Woods, Geoffrey Corlett

(56) References cited:
- EP-A- 0 267 778
- EP-A- 0 359 305

## Description

This invention relates to a process for the preparation of fungicidally active cyclopentane derivatives.

EP-A2-0267778 discloses fungicidally active cyclopentane derivatives of the general formula 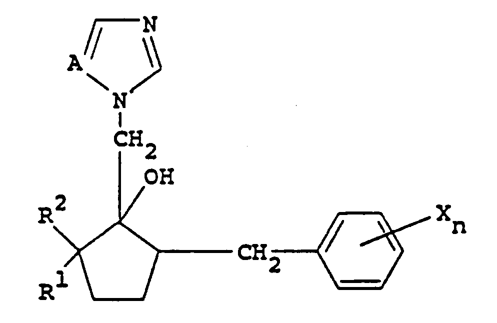
or acid addition salts or metal complexes thereof, in which R¹ and R² each independently represents a C₁₋₅alkyl group or a hydrogen atom, each X represents a halogen atom, a C₁₋₅alkyl group or a phenyl group, n represents 0, 1 or 2 and A represents a nitrogen atom or CH, provided that R¹ is not a hydrogen atom when R² is a hydrogen atom.

GB-A1-2180236 discloses fungicidally active cyclopentane derivatives of the above general formula in which R¹ and R² both represent hydrogen atoms, n represents an integer from 1 to 5, each X represents a hydrogen atom, a halogen atom, an alkyl group, a haloalkyl group, a phenyl group, a cyano group or a nitro group and A represents a nitrogen atom or a CH group.

The compounds disclosed in EP-A2-0267778 and GB-A1-2180236 exist in two stereoisomeric forms which have the following structures:- 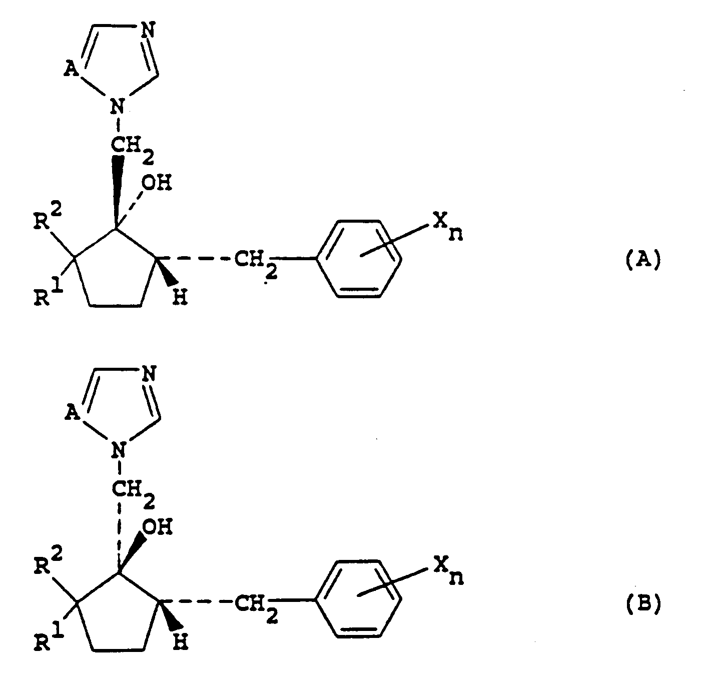
The letters A and B will be used hereinafter to denote compounds having the same stereochemical configuration as isomers A and B above.

Isomers A and B can be separated by, for instance, chromatography and exhibit different fungicidal activity. Generally, isomers of formula A exhibit greater fungicidal activity than isomers of formula B.

The processes fcr the preparation of the above compounds disclosed in GB-A1-2180236 and EP-A2-0267778 produce a mixture of isomers of formula A and B thereby necessitating the inclusion of an isomer separation step in the process. Moreover, production of the more active isomer A is not maximised in such processes because a portion of the starting materials is consumed in the simultaneous generation of the less active isomer B. However, a new process has now been discovered for the preparation of such compounds which is highly stereospecific in favour of the more active isomer A.

According to the present invention there is therefore provided a process for the preparation of a compound of the general formula 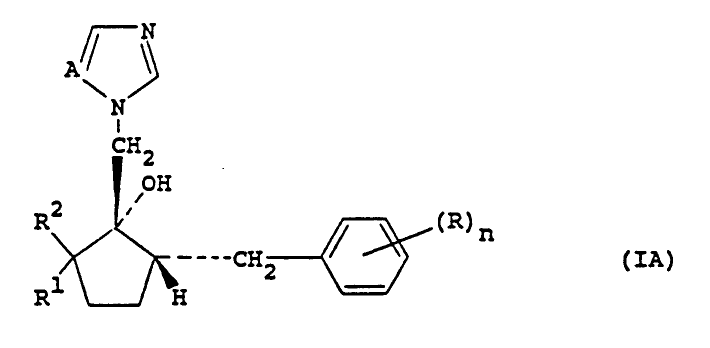
or an acid addition salt or metal complex thereof, in which n represents an integer from 0 to 5, each R represents a halogen atom, nitro, cyano, alkyl, haloalkyl or phenyl group, R¹ and R² independently represent a hydrogen atom or an alkyl group, and A represents a nitrogen atom or a CH group; which comprises reacting a compound of the general formula 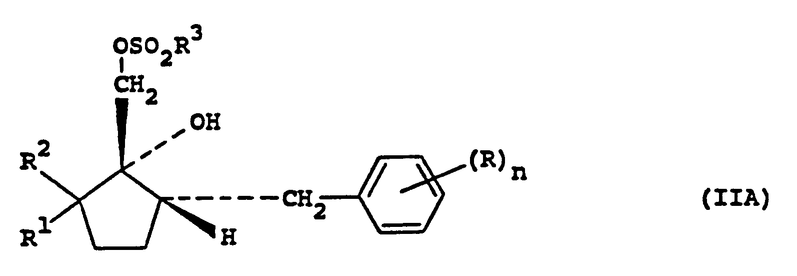
in which n, R, R¹ and R² are as defined above and R³ represents an optionally substituted alkyl or aryl group, preferably a C₁₋₄alkyl group or a phenyl group each optionally substituted by one or more substituents selected from halogen atoms, nitro, cyano, hydroxyl, C₁₋₄alkyl, C₁₋₄haloalkyl, C₁₋₄alkoxy, C₁₋₄haloalkoxy, amino, C₁₋₄alkylamino, di-C₁₋₄alkylamino, C₁₋₄alkoxycarbonyl, carboxyl, C₁₋₄alkanoyl, C₁₋₄alkylthio, C₁₋₄alkylsulphinyl, C₁₋₄alkylsulphonyl, carbamoyl, C₁₋₄alkylamido, C₃₋₈cycloalkyl and phenyl groups; with a compound of the general formula 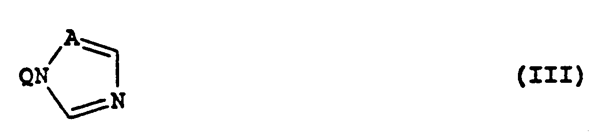
in which A is as defined above and Q represents a hydrogen or alkali metal, preferably sodium, atom, in the presence of a base; and, optionally, converting the resulting compound of formula IA into an acid addition salt or metal complex thereof.

It is preferred that the base is an inorganic base, such as potassium carbonate, potassium hydroxide, sodium carbonate and sodiumhydroxide. Preferably, two equivalents of base are used.

The process may be carried out in the presence of a solvent. Suitable solvents include N-methylpyrrolidone, acetonitrile, dimethylformamide, dimethylsulphoxide and high boiling ketones, such as methyl isobutyl ketone.

The reaction is suitably carried out at a temperature from 80° to 170°C, depending on the nature of the solvent, if present. The preferred temperature is from 100° to 150°C.

When any of the substituents R, R¹ and R² represents or contains an alkyl substituent group, this may be linear or branched and may contain up to 12, preferably up to 6, and especially up to 4, carbon atoms. A cycloalkyl substituent group may contain 3 to 8, preferably 3 to 6, carbon atoms.

It is preferred that R¹ and R² independently represent a hydrogen atom or a C₁₋₄alkyl, particularly a methyl, group.

Preferably, R represents a halogen, especially a chlorine, atom.

It is particularly preferred that n is 1, R represents a chlorine atom, preferably substituted at the 4-position of the phenyl ring, R¹ and R² both represent a hydrogen atom or both represent a methyl group and A represents a nitrogen atom.

Compounds of formula IIA may be conveniently prepared by reacting a compound of the general formula 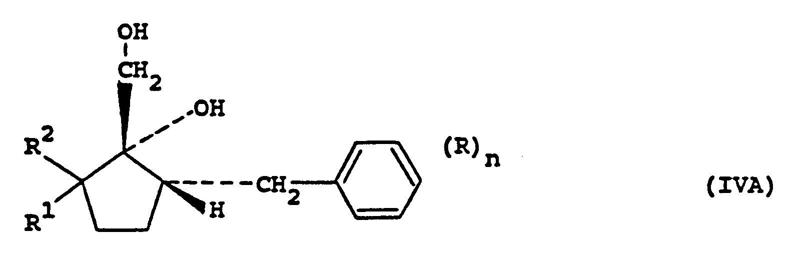
in which n, R, R¹ and R² are as defined above, with a compound of the general formula

R³SO₂X (V)

in which R³ is as defined above and X represents a halogen, preferably a chlorine or bromine, atom in the presence of a base, such as triethylamine. The compounds of formula IIA and a process for their preparation form the subject of EP-A-359 305.

Compounds of formula IVA may be conveniently prepared either by reacting a compound of the general formula 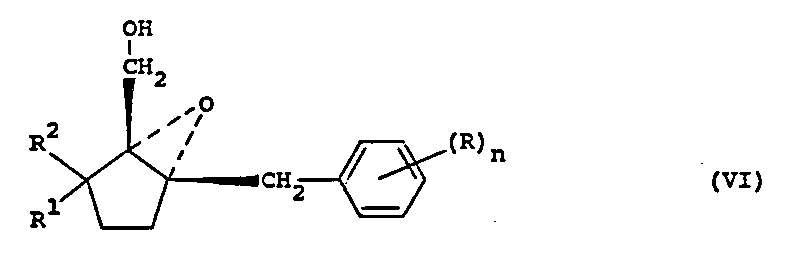
in which n, R, R¹ and R² are as defined above, with a suitable reducing agent, for instance, a complex metal hydride, such as lithium aluminium hydride, at a temperature from 35°C to reflux temperature; or by reacting a compound of the general formula 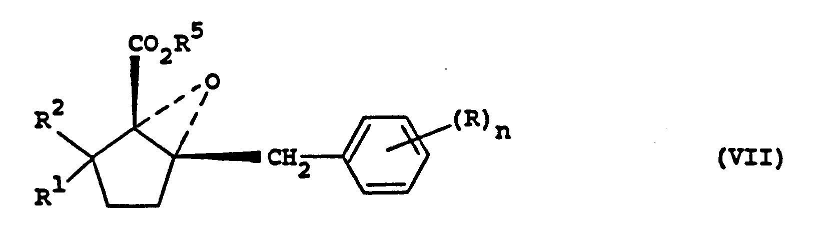
in which n, R, R¹ and R² are as defined above and R⁵ represents a hydrogen atom or an alkyl, preferably C₁₋₄alkyl, group, with a reducing agent, such as lithium aluminium hydride, at a temperature from 35°C to reflux temperature. These reactions are stereospecific due to the presence of the epoxy group in the starting materials of formula VI and formula VII, and, under the conditions specified, compounds of formula IVA will be produced. The compounds of formula IVA and a process for their preparation form the subject of EP-A-359 305.

Compounds of formula VI may be conveniently prepared by reacting a compound of formula VII, as defined above, with a reducing agent, such as lithium aluminium hydride, preferably at a temperature from room temperature to 85°C. The compounds of formula VI and a process for their preparation form the subject of EP-A-359 305.

Compounds of formula VII may be conveniently prepared by reacting a compound of the general formula 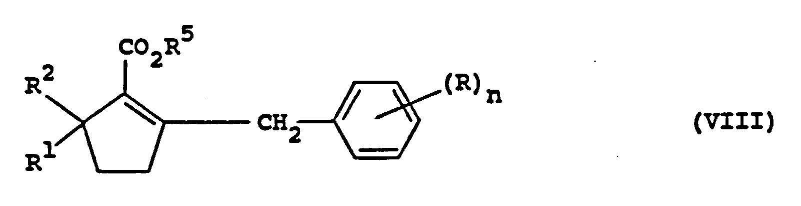
in which n, R, R¹, R² and R⁵ are as defined above, with a peracid, such as peracetic acid, perbenzoic acid or perphthalic acid. The compounds of formula VII and a process for their preparation form the subject of EP-A-361 560.

Compounds of formula VIII may be conveniently prepared by heating a compound of the general formula 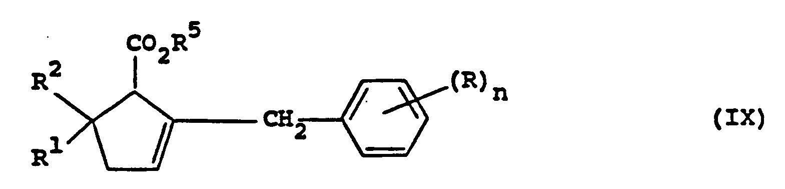
or by reacting a compound of the general formula 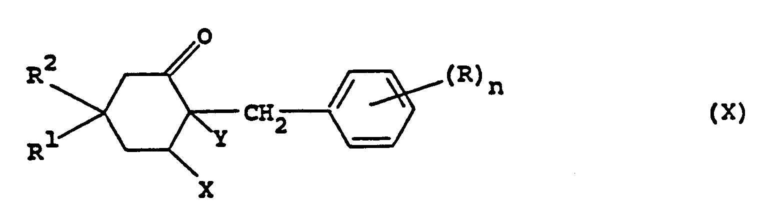
in which n, R, R¹, R² and R⁵ are as defined above and X and Y independently represent a halogen, preferably a chlorine or bromine, atom, with a compound of the general formula

MOR⁵ (XI)

in which R⁵ is as defined above and M represents an alkali metal, preferably a sodium, atom, in the presence of a polar solvent. It is preferred that the polar solvent is a compound of the general formula

R⁵OH (XII)

in which R⁵ is as defined above. Preferably, R⁵ has the same meaning in formula XI and formula XII. For instance, if the compound of formula XI is sodium methoxide, it is preferred that the solvent of formula XII is methanol. The compounds of formula VIII and a process for their preparation form the subject of EP-A-358 259.

Compounds of formula IX may be conveniently prepared by reacting a compound of formula X, as defined above, with a compound of formula XI, as defined above, in the presence of a solvent XII, as defined above, preferably at a temperature in the range of 0°-20°C. The compounds of formula IX and a process for their preparation form the subject of EP-A-358 259.

Compounds of formula X may be conveniently prepared by reacting a compound of the general formula 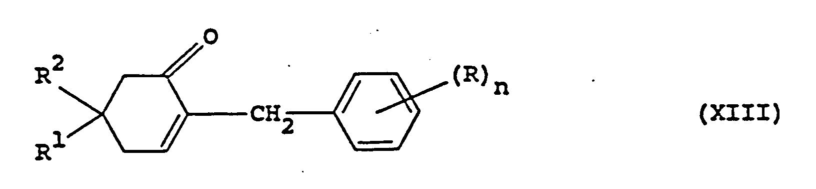
in which n, R, R¹ and R² are as defined above, with a compound XY, in which X and Y are as defined above. Alternatively, compounds of formula X may be generated in situ and then heated with a compound of formula XI in the presence of a solvent of formula XII as described above to form compounds of formula VIII in a one-pot synthesis. The compounds of formula X and a process for their preparation form the subject of EP-A-358 259.

Compounds of formula XIII may be conveniently prepared by reacting a compound of the general formula 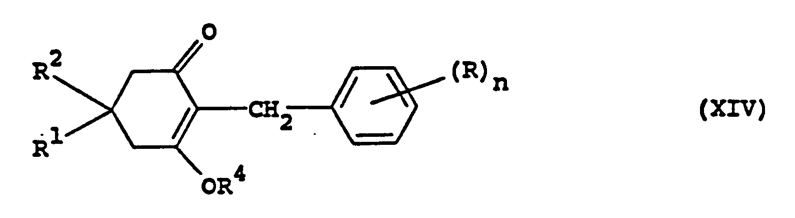
in which n, R, R¹ and R² are as defined above and R⁴ represents an alkyl, preferably a C₁₋₄alkyl, group, with a suitable reducing agent, for instance, a complex metal hydride, such as lithium aluminium hydride or sodium aluminium hydride, or hydrogen in combination with a catalyst, and subsequently hydrolysing the reaction mixture. The compounds of formula XIII and a process for their preparation form the subject of EP-A-358 259.

Compounds of formula XIV may be conveniently prepared by reacting a compound of the general formula 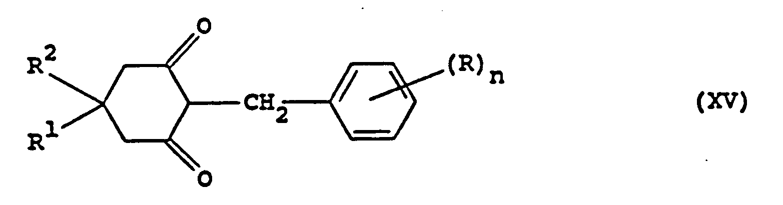
in which n, R, R¹ and R² are as defined above, with a compound of the general formula

R⁴OH (XVI)

in which R⁴ is as defined above, in the presence of an acid, such as sulphuric acid, p-toluenesulphonic acid or an ion exchange resin. The compounds of formula XIV and a process for their preparation form the subject of EP-A-358 259.

Compounds of formula XV may be conveniently prepared by reacting a compound of the general formula 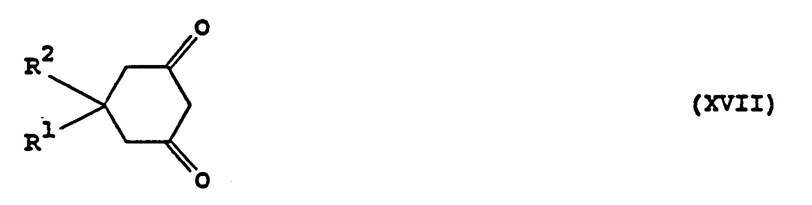
in which R¹ and R² are as defined above, with a compound of the general formula 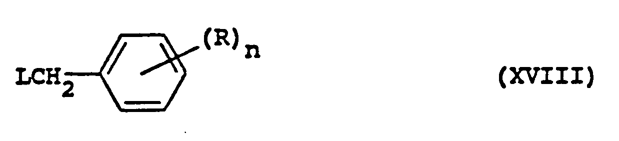
in which R and n are as defined above and L represents a suitable leaving group, in the presence of a suitable base, such as potassium hydroxide. The compounds of formula XV form the subject of EP-A-358 259.

Compounds of formula III, V, XI, XII, XVI, XVII and XVIII and the compounds XY are known compounds or can be prepared by processes analogous to known processes.

The overall process used to synthesise compounds of formula IA starting from compunds of formula XVII and formula XVIII, as described above, is set out in the following reaction scheme:- 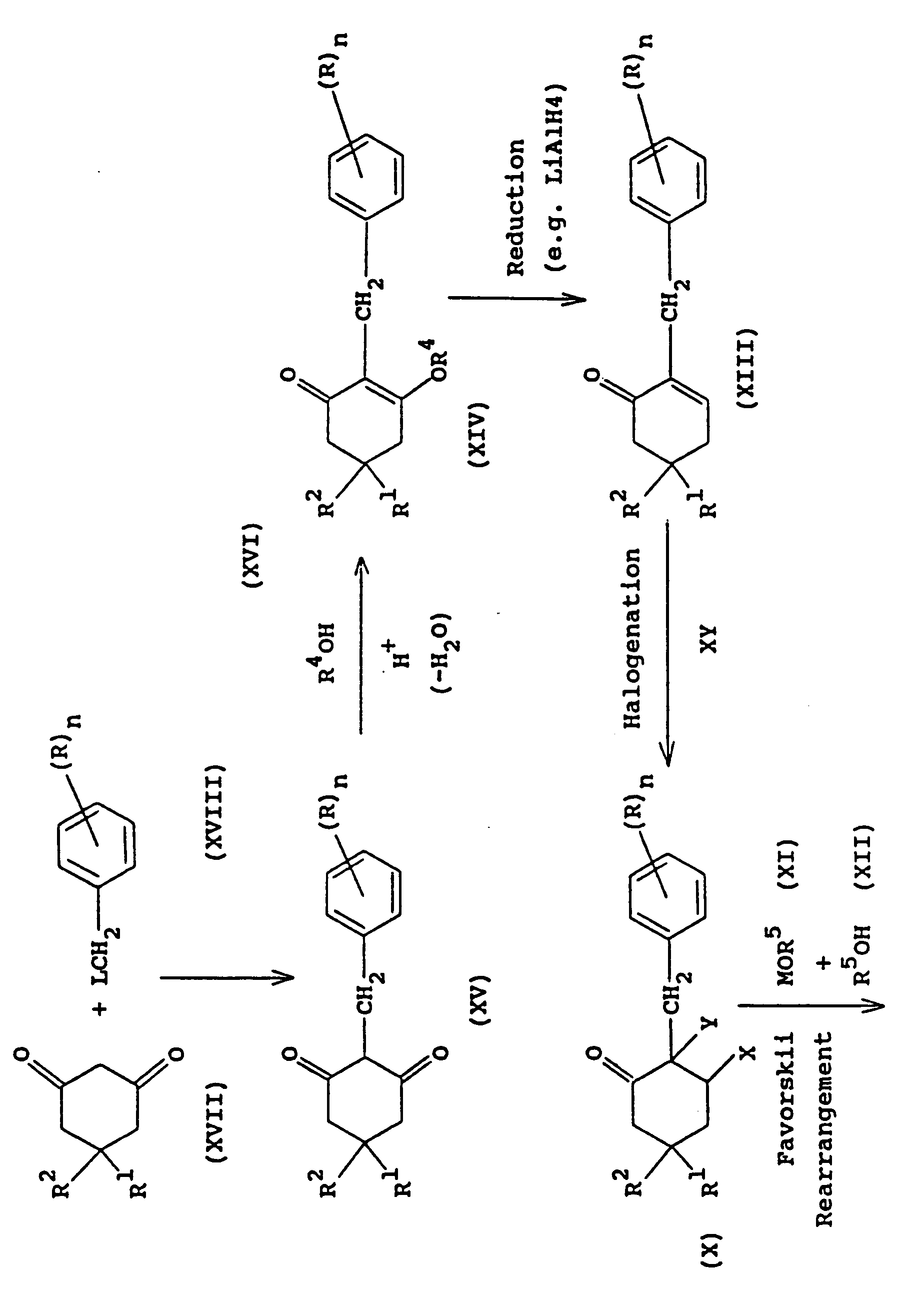 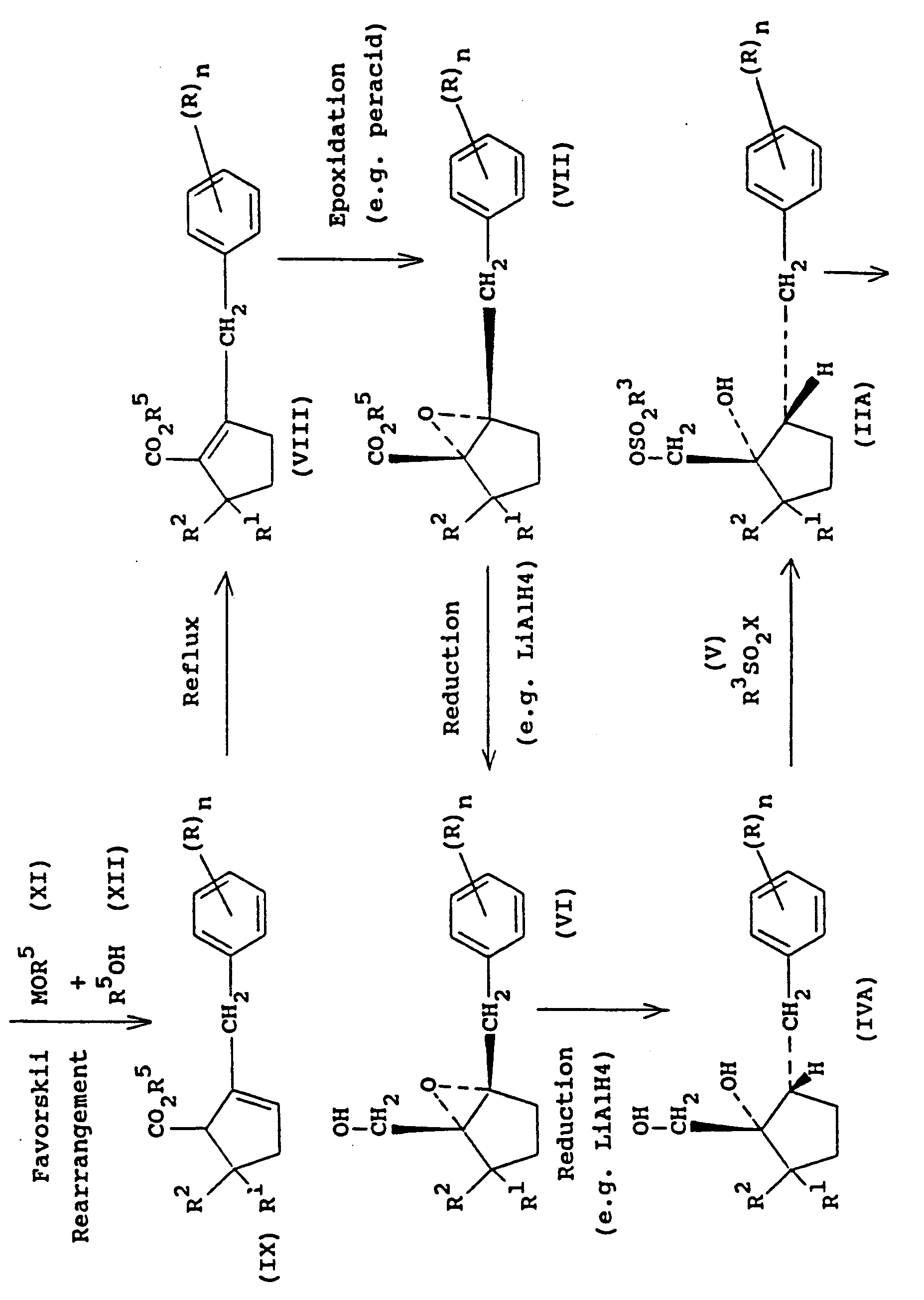 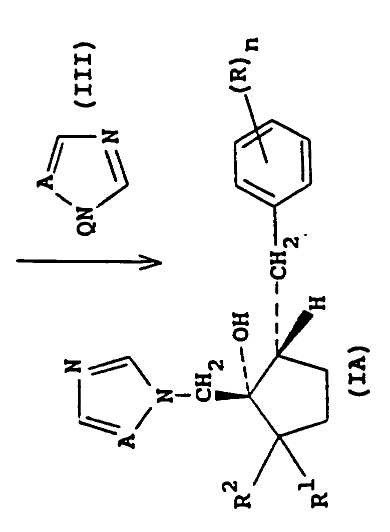
In the above reaction scheme, n, R, R¹, R², R³, R⁴, R⁵, X, Y, L, M, Q and A are as previously defined.

The invention is further illustrated by the following Examples.

### Example 1

### Preparation of 1-(4-chlorobenzyl)-3,3-dimethyl-2-hydroxy-2-(1,2,4-triazol-1-yl)methylcyclopentane (Isomer A) (n=1, R=4-Cl, R¹=R²=CH₃, A=N)

### (a) Preparation of 2-(4-chlorobenzyl)-5,5-dimethylcyclohexane-1,3-dione

449g (3.21 mols) dimedone (5,5-dimethylcyclohexane-1,3-dione) were added to a solution of aqueous potassium hydroxide comprising 166g of 85% potassium hydroxide (2.52 moles) in 700 ml of water. The mixture was then warmed and a clear orange solution was obtained at 47°C. The solution was then heated to 59°C and 544g (3.21 mols) molten 4-chlorobenzyl chloride were added over a period of 1 hour with further heating to 85°C. Heating was continued for a further 2½ to 3 hours up to a temperature of 100°C. The mixture was then cooled, the solid product filtered off, washed with water and dried in a vacuum oven at 50°C. The crude solid (815g) was then dissolved in 2400ml methanol at reflux and 200ml water added to produce a permanent cloudiness. The mixture was then allowed to cool to room temperature overnight with stirring. The solid so obtained was filtered, washed with about 400ml cold methanol and dried in a vacuum oven to produce 340g 2-(4-chlorobenzyl)-5,5-dimethylcyclohexane-1,3-dione as a white solid, m.pt. 188-190°C. Yield: 42%.

### (b) Preparation of 2-(4-chlorobenzyl)-3-(2-methylpropoxy)-5,5-dimethylcyclohex-2-en-1-one

325g (1.23 mol) of the 2-(4-chlorobenzyl)-5,5-dimethylcyclohexane-1,3-dione obtained in (a), 1.6 litres toluene, 182g (2.5 mol) isobutanol and 5g p-toluenesulphonic acid were stirred together at reflux under a Dean-Stark apparatus. The temperature of the reaction mixture was approximately 90°C. As water distilled off, the reaction mixture changed from a thin slurry to a yellow solution. After 14 hours reflux, the reaction mixture was cooled and shaken twice with 500ml aliquots of 10% aqueous sodium hydroxide. The toluene layer was then flashed to give 389g yellow/orange oil which crystallised on standing. Recrystallisation of the solid from 60/80 petroleum produced 331g 2-(4-chlorobenzyl)-3-(2-methylpropoxy)-5,5-dimethylcyclohex-2-en-1-one as a white crystalline solid, m.pt. 60-61°C. Yield: 84%.

### (c) Preparation of 2-(4-chlorobenzyl)-3-methoxy-5,5-dimethylcyclohex-2-en-1-one

A solution of 154g of the 2-(4-chlorobenzyl)-3-(2-methylpropoxy)-5,5-dimethylcyclohex-2-en-1-one obtained in Example 1 in 1200 ml methanol containing 3g p-toluenesulphonic acid was refluxed for 2 hours. The reaction mixture was then extracted with 3 litres water and 1 litre diethyl ether and re-extracted with a further 1 litre diethyl ether. The organic phases were then back-washed first with 200 ml 10% aqueous sodium hydroxide and then with 100 ml saturated sodium chloride solution, dried over anhydrous magnesium sulphate and flashed. The residue was then crystallised in 60/80 petroleum, filtered and air-dried to give 98g 2-(4-chlorobenzyl)-3-methoxy-5,5-dimethylcyclohex-2-en-1-one as a white solid, m.pt. 62-63°C. Yield: 73%.

### (d) Preparation of 2-(4-chlorobenzyl)-5,5-dimethylcyclohex-2-en-1-one

98g (0.35 mol) of the 2-(4-chlorobenzyl)-3-methoxy-5,5-dimethylcyclohex-2-en-1-one obtained in (c) were added to a slurry of 6.65g (0.175 mol) lithium aluminium hydride in 490 mls diethyl ether at a rate sufficient to maintain reflux and the final reaction mixture refluxed for a further 30 minutes. 5ml water were then added, followed by 5ml 15% aqueous sodium hydroxide and a further 15ml water and the resulting precipitate was filtered off. The filtrate was then shaken in 200ml 5M hydrochloric acid for five minutes and the organic layer then separated, washed twice with 100ml aliquots of saturated sodium bicarbonate solution, dried over anhydrous magnesium sulphate and stripped. The resulting oil was then dissolved in 430mls dichloromethane, 18g (0.085 mols) pyridinium chlorochromate were added and the reaction mixture stirred for 3 hours. 600ml diethyl ether were added and the solid was then filtered off. The filtrate was washed three times with 10% sodium hydroxide, once with 2.5M hydrochloric acid and once with saturated sodium bicarbonate solution. It was then dried over anhydrous magnesium sulphate and stripped to give 82g of crude product. Distillation of the crude product under reduced pressure (0.15mm mercury) gave 79g 2-(4-chlorobenzyl)-5,5-dimethylcyclohex-2-en-1-one, b.pt. 130°C at 0.15mm mercury. Yield: 91%.

### (e) Preparation of 2-(4-chlorobenzyl)-2,3-dibromo-5,5-dimethylcyclohexan-1-one

10g (40.2 mmol) cf the 2-(4-chlorobenzyl)-5,5-dimethylcyclohex-2-en-1-one obtained in (d) were dissolved in 50ml 30/40 petroleum at 0°C. 6.72g (40.2 mmol) bromine were then added to the solution. After 5-10 minutes, the solution decolourised and a precipitate formed. The solution was then cooled further and the precipitate filtered off to give 12.4g 2-(4-chlorobenzyl)-2,3-dibromo-5,5-dimethylcyclohexan-1-one as a solid, m.pt. 82-84°C. Yield: 75%.

### (f) Preparation of 1-(4-chlorobenzyl)-3,3-dimethyl-2-methoxycarbonylcyclopent-1-ene

A solution of sodium methoxide was prepared by adding 2.8g (121 mmol) sodium to 50ml methanol. A slurry of the 2-(4-chlorobenzyl)-2,3-dibrcmo-5,5-dimethylcyclohexan-1-one obtained in (e) in methanol was then prepared and added to the sodium methoxide solution at reflux. Reflux was continued overnight. The reaction mixture was then quenched with 200ml water, extracted twice with 100ml aliquots of diethyl ether, backwashed with water, dried over anhydrous magnesium sulphate and flashed to give 8g of a yellow oil. By gas chromatography analysis, it was established that 6.6g 1-(4-chlorobenzyl)-3,3-dimethyl-2-methoxycarbonyl-cyclopent-1-ene were produced as an oil. The structure of the product was established by n.m.r. spectroscopy. Yield: 78%.

### (g) Preparation of 1-(4-chlorobenzyl)-1,2-epoxy-3,3-dimethyl-2-methoxycarbonylcyclopentane

23.5g (3 equivalents) "PROXITANE 4002" (Trade Mark: 36-40% (w/w) peracetic acid in acetic acid) were added to 9.8g (35.1 mmol) 1-(4-chlorobenzyl)-3,3-dimethyl-2-methoxycarbonylcyclopent-1-ene prepared as described in (f) in 90ml trichloromethane. The resulting mixture was refluxed for 3 hours and then extracted twice with 50ml aliquots of trichloromethane, backwashed once with 50ml dilute sodium bicarbonate solution and twice with 50ml aliquots of saturated sodium metabisulphite, washed with saturated sodium chloride solution, dried over anhydrous magnesium sulphate and flashed to give 12g of a pale yellow oil which crystallised on cooling. Trituration in 30/40 petroleum gave 5.8g 1-(4-chlorobenzyl)-1,2-epoxy-3,3-dimethyl-2-methoxycarbonylcyclopentane as a white crystalline solid, m.pt. 86-87°C. Yield: 56%.

### (h) Preparation of 1-(4-chlorobenzyl)-3,3-dimethyl-2-hydroxy-2-hydroxymethylcyclopentane (Isomer A)

2.7g (71 mmol) lithium aluminium hydride were added to a suspension of 2.7g (24 mmol) aluminium chloride partially dissolved in 120 ml 1,2-dimethoxyethane, the addition of lithium aluminium hydride causing a rise in temperature to 50°C. The resulting slurry was incubated at 50°C for 30 minutes. A solution of 6.8g (23 mmol) 1-(4-chlorobenzyl)-1,2-epoxy-3,3-dimethyl-2-methoxycarbonylcyclopentane prepared as described in (g) in 30ml 1,2-dimethoxyethane was then added over a period of 30 minutes whilst maintaining the temperature of the reaction mixture at 50-55°C. After 1 hour, thin layer chromatography indicated that the reaction was complete and the excess lithium aluminium hydride was therefore destroyed by adding 10ml saturated ammonium chloride solution followed by 10ml water. This produced a sludgy solid which filtered only slowly. After filtration, the solid was dissolved in 2N hydrochloric acid and extracted twice with diethyl ether. The extracts were combined with the filtrate and flashed to give 5.9g crude product. Trituration with cold 60/80 petroleum and a little diethyl ether gave 5.45g 1-(4-chlorobenzyl)-3,3-dimethyl-2-hydroxy-2-hydroxymethylcyclopentane as a white solid, m.pt. 103-104°C. Yield: 82%.

### (i) Preparation of 1-(4-chlorobenzyl)- 3,3-dimethyl-2-hydroxy-2-methylsulphonyloxymethylcyclopentane (Isomer A)

2.76g (1.05 equivalents) methylsulphonyl chloride were added to a solution of 6.42g (24 mmol) 1-(4-chlorobenzyl)-3,3-dimethyl-2-hydroxy-2-hydroxymethylcyclopentane prepared as described in (h) and 2.55g (1.1 equivalents) triethylamine in 50ml dichloromethane at 10-15°C. The reaction mixture was kept at 10-15°C (for a further 2 hours and then washed with 20 ml water, then 20ml aqueous sodium bicarbonate solution, dried over anhydrous magnesium sulphate and flashed. Trituration with 60/80 petroleum gave 6.64g 1-(4-chlorobenzyl)-3,3-dimethyl-2-hydroxy-2-methylsulphonyloxymethylcyclopentane (Isomer A) as a white solid, m.pt. 113-114°C. Yield: 80%.

### (j) Preparation of 1-(4-chlorobenzyl)-3,3-dimethyl-2-hydroxy-2-(1,2,4-triazol-1-yl)methylcyclopentane (Isomer A)

A mixture of 0.22g 1-(4-chlorobenzyl)-3,3-dimethyl-2-hydroxy-2-methylsulphonyloxymethylcyclopentane prepared as described in (i), 47 mg 1,2,4-triazole and 0.1g potassium carbonate in 3 ml N-methylpyrrolidone was warmed at 50°C for 2 hours. A further 0.1g potassium carbonate was then added, the temperature was raised to 120°C and the reaction continued overnight at this temperature. The reaction mixture was then partitioned between diethyl ether and water and the organic solution flashed to give 1-(4-chlorobenzyl)-3,3-dimethyl-2-hydroxy-2-(1,2,4-triazol-1-yl)methylcyclopentane crystallised from petroleum as a single stereoisomer (Isomer A). The structure of the product was established by n.m.r. spectroscopy. Purity: 90 %.

### Example 2

### Preparation of 1-(4-chlorobenzyl)-3,3-dimethyl-2-hydroxy-2-(1,2,4-triazol-1-yl)methylcyclopentane (Isomer A) (n=1, R=4-Cl, R¹=R²=CH₃, A=N)

### a) Preparation of 2-(4-chlorobenzyl)-2,3-dibromo-5,5-dimethylcyclohexan-1-one

5g of the 2-(4-chlorobenzyl)-5,5-dimethylcyclohex-2-en-1-one obtained in Example 1 (d) were dissolved in 25ml tetrachloromethane at 5-10°C. 3.2g bromine were then added to the solution over a period of 10 minutes. The solution decolourised and 2-(4-chlorobenzyl)-2,3-dibromo-5,5-dimethylcyclohexan-1-one formed in solution.

### (b) Preparation of 2-(4-chlorobenzyl)-4,4-dimethyl-3-methoxycarbonylcyclopent-1-ene

A solution of sodium methoxide was prepared by adding 1.1g sodium to 10ml methanol. 50ml methanol was then added to the reaction mixture obtained in (a) followed by the solution of sodium methoxide, keeping the temperature of the reaction mixture at 10-15°C. After 2 hours, the mixture was partitioned between dichloromethane and water, backwashed with water, dried over anhydrous magnesium sulphate and flashed to give 5.65g crude 2-(4-chlorobenzyl)-4,4-dimethyl-3-methoxycarbonylcyclopent-1-ene as an oil. The structure of the product was established by n.m.r. spectroscopy.

### (c) Preparation of 1-(4-chlorobenzyl)-3,3-dimethyl-2-methoxycarbonylcyclopent-1-ene

The crude 2-(4-chlorobenzyl)-4,4-dimethyl-3-methoxycarbonylcyclopent-1-ene obtained in (b) was refluxed for 15 hours in methanol as solvent containing one equivalent of sodium methoxide to give 1-(4-chlorobenzyl)-3,3-dimethyl-2-methoxycarbonylcyclopent-1-ene as an oil.

### (d) Preparation of 1-(4-chlorobenzyl)-1,2-epoxy-3,3-dimethyl-2-methoxycarbonylcyclopentane

23.5g (3 equivalents) "PROXITANE 4002" (Trade Mark: 36-40% (w/w) peracetic acid in acetic acid) were added to 9.8g (35.1 mmol) 1-(4-chlorobenzyl)-3,3-dimethyl-2-methoxycarbonylcyclopent-1-ene prepared as described in (c) in 90ml trichloromethane. The resulting mixture was refluxed for 3 hours and then extracted twice with 50ml aliquots of trichloromethane, backwashed once with 50ml dilute sodium bicarbonate solution and twice with 50ml aliquots of saturated sodium metabisulphite, washed with saturated sodium chloride solution, dried over anhydrous magnesium sulphate and flashed to give 12g of a pale yellow oil which crystallised on cooling. Trituration in 30/40 petroleum gave 5.8g 1-(4-chlorobenzyl)-1,2-epoxy-3,3-dimethyl-2-methoxycarbonylcyclopentane as a white crystalline solid, m.pt. 86-87°C. Yield: 56%.

### (e) Preparation of 1-(4-chlorobenzyl)-3,3-dimethyl-2-hydroxy-2-hydroxymethylcyclopentane (Isomer A)

2.7g (71 mmol) lithium aluminium hydride were added to a suspension of 2.7g (24 mmol) aluminium chloride partially dissolved in 120 ml 1,2-dimethoxyethane, the addition of lithium aluminium hydride causing a rise in temperature to 50°C. The resulting slurry was incubated at 50°C for 30 minutes. A solution of 6.8g (23 mmol) 1-(4-chlorobenzyl)-1,2-epoxy-3,3-dimethyl-2-methoxycarbonylcyclopentane prepared as described in (d) in 30ml 1,2-dimethoxyethane was then added over a period of 30 minutes whilst maintaining the temperature of the reaction mixture at 50-55°C. After 1 hour, thin layer chromatography indicated that the reaction was complete and the excess lithium aluminium hydride was therefore destroyed by adding 10ml saturated ammonium chloride solution followed by 10ml water. This produced a sludgy solid which filtered only slowly. After filtration, the solid was dissolved in 2N hydrochloric acid and extracted twice with diethyl ether. The extracts were combined with the filtrate and flashed to give 5.9g crude product. Trituration with cold 60/80 petroleum and a little diethyl ether gave 5.45g 1-(4-chlorobenzyl)-3,3-dimethyl-2-hydroxy-2-hydroxymethylcyclopentane as a white solid, m.pt. 103-104°C. Yield: 82%

### (f) Preparation of 1-(4-chlorobenzyl)- 3,3-dimethyl-2-hydroxy-2-methylsulphonyloxymethylcyclopentane (Isomer A)

2.76g (1.05 equivalents) methylsulphonyl chloride were added to a solution of 6.42g (24 mmol) 1-(4-chlorobenzyl)-3,3-dimethyl-2-hydroxy-2-hydroxymethylcyclopentane prepared as described in (e) and 2.55g (1.1 equivalents) triethylamine in 50ml dichloromethane at 10-15°C. The reaction mixture was kept at 10-15° (for a further 2 hours and then washed with 20 ml water, then 20ml aqueous sodium bicarbonate solution, dried over anhydrous magnesium sulphate and flashed. Trituration with 60/80 petroleum gave 6.64g 1-(4-chlorobenzyl)-3,3-dimethyl-2-hydroxy-2-methylsulphonyloxymethylcyclopentane (Isomer A) as a white solid, m.pt. 113-114°C. Yield: 80%.

### (g) Preparation of 1-(4-chlorobenzyl)-3,3-dimethyl-2-hydroxy-2-(1,2,4-triazol-1-yl)methylcyclopentane (Isomer A)

A mixture of 0.22g 1-(4-chlorobenzyl)-3,3-dimethyl-2-hydroxy-2-methylsulphonyloxymethylcyclopentane prepared as described in (f), 47 mg 1,2,4-triazole and 0.1g potassium carbonate in 3 ml N-methylpyrrolidone was warmed at 50°C for 2 hours. A further 0.1g potassium carbonate was then added, the temperature was raised to 120°C and the reaction continued overnight at this temperature. The reaction mixture was then partitioned between diethyl ether and water and the organic solution flashed to give 1-(4-chlorobenzyl)-3,3-dimethyl-2-hydroxy-2-(1,2,4-triazol-1-yl)methylcyclo pentane crystallised from petroleum as a single stereoisomer (Isomer A). The structure of the product was established by n.m.r. spectroscopy. Purity: 90 %

## Claims

1. A process for the preparation of a compound of the general formula 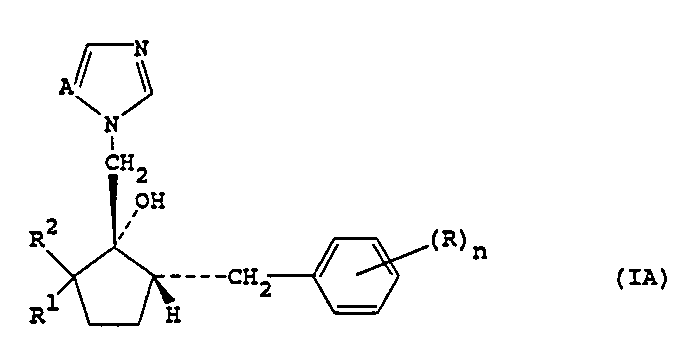 or an acid addition salt or metal complex thereof, in which n represents an integer from 0 to 5, each R represents a halogen atom, nitro, cyano, alkyl, haloalkyl or phenyl group; R¹ and R² independently represent a hydrogen atom or an alkyl group; and A represents a nitrogen atom or a CH group; which comprises reacting a compound of the general formula 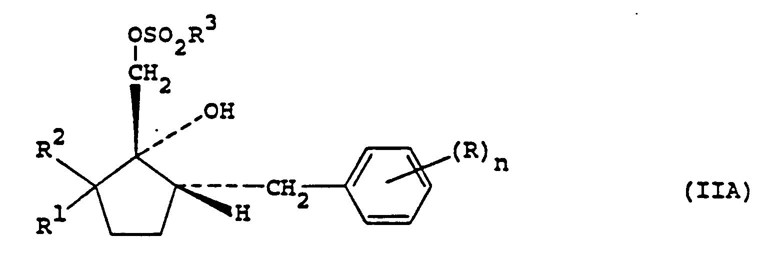 in which n ,R, R¹ and R² are as defined above and R³ represents an optionally substituted alkyl or aryl group; with a compound of the general formula 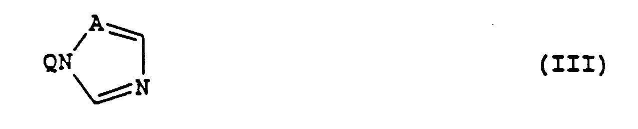 in which A is as defined above and Q represents a hydrogen or alkali metal atom, in the presence of a base; and, optionally, converting the resulting compound of formula IA into an acid addition salt or metal complex thereof.

2. A process according to claim 1 in which the compound of formula IIA is prepared by reacting a compound of the general formula 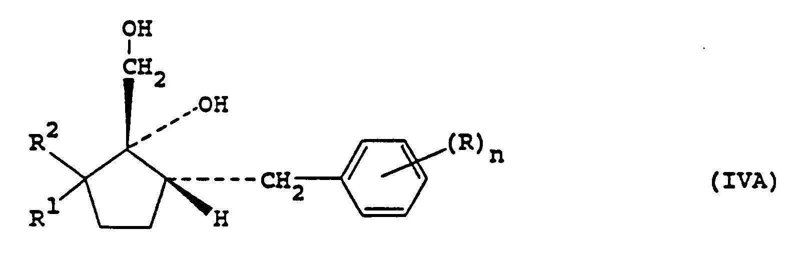 in which n, R, R¹ and R² are as defined in claim 1, with a compound of the general formula
R³SO₂X (V)
in which R³ is as defined in claim 1 and X represents a halogen atom, in the presence of a base.

3. A process according to claim 2 in which the compound of formula IVA is prepared either by
(a) reacting a compound of the general formula 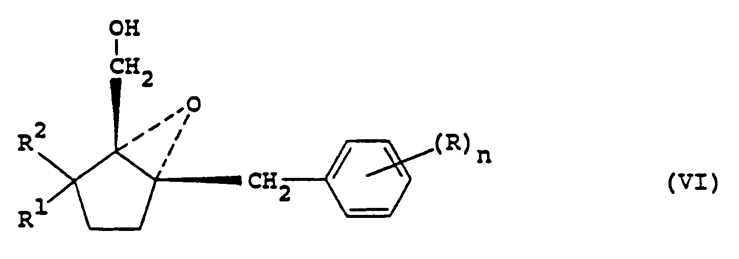 in which n, R, R¹ and R² are as defined in claim 1, with a reducing agent at a temperature from 35°C to reflux temperature; or
(b) reacting a compound of the general formula 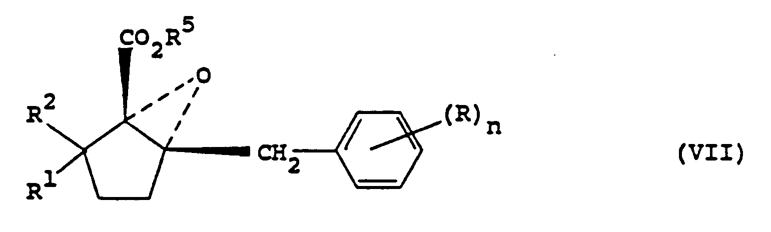 in which n, R, R¹ and R² are as defined in claim 1 and R⁵ represents a hydrogen atom or an alkyl group, with a reducing agent at a temperature from 35°C to reflux temperature.

4. A process according to claim 3 in which the compound of formula VI is prepared by reacting a compound of formula VII, as defined in claim 3, with a reducing agent at a temperature from room temperature to 85°C.

5. A process according to claim 3 or claim 4 in which the compound of formula VII is prepared by reacting a compound of the general formula 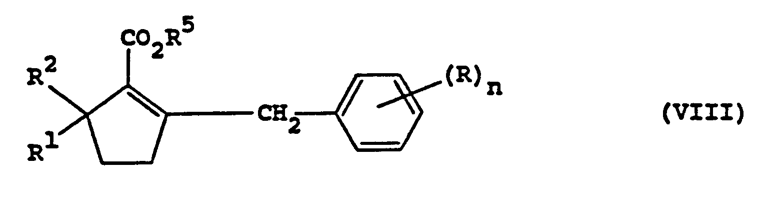 in which n, R, R¹ and R² are as defined in claim 1 and R⁵ is as defined in claim 3, with a peracid.

6. A process according to claim 5 in which the compound of formula VIII is prepared by heating a compound of the general formula 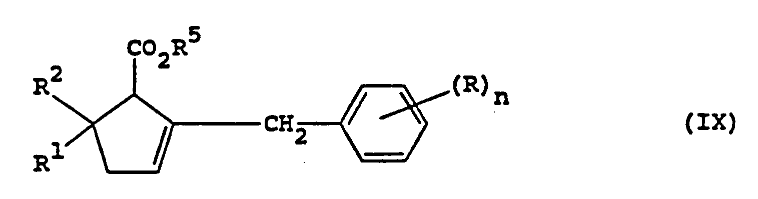 or by reacting a compound of the general formula 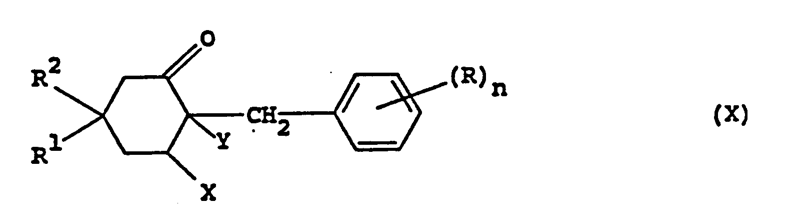 in which n, R, R¹ and R² are as defined in claim 1, R⁵ is as defined in claim 3, X is as defined in claim 2 and Y represents a halogen atom, with a compound of the general formula
MOR⁵ (XI)
in which R⁵ is as defined in claim 3 and M represents an alkali metal atom, in the presence of a polar solvent.

7. A process according to claim 6 in which the compound of formula IX is prepared by reacting a compound of formula X, as defined in claim 6, with a compound of formula XI, as defined in claim 6, in the presence of a solvent of formula (XII):
R⁵OH (XII)
wherein R⁵ is as defined in claim 3.

8. A process according to claim 6 or claim 7 in which the compound of formula X is prepared by reacting a compound of the general formula 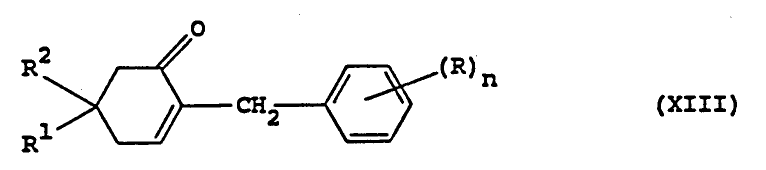 in which n, R, R¹ and R² are as defined in claim 1, with a compound XY in which X is as defined in claim 2 and Y is as defined in claim 6.

9. A process according to claim 8 in which the compound of formula XIII is prepared by reacting a compound of the general formula in which n, R, R¹ and R² are as defined in claim 1 and R⁴ represents an alkyl group, with a reducing agent, and subsequently hydrolysing the reaction mixture.

10. A process according to claim 9 in which the compound of formula XIV is prepared by reacting a compound of the general formula 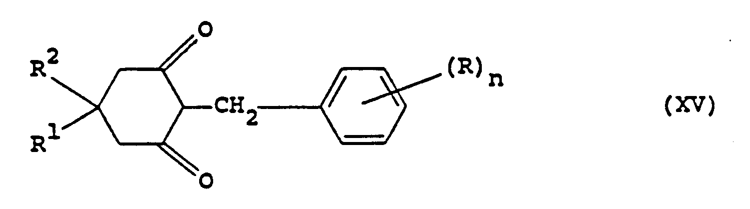 in which n, R, R¹ and R² are as defined in claim 1, with a compound of the general formula
R⁴OH (XVI)
in which R⁴ is as defined in claim 9, in the presence of an acid

11. A process according to claim 10 in which the compound of formula XV is prepared by reacting a compound of the general formula 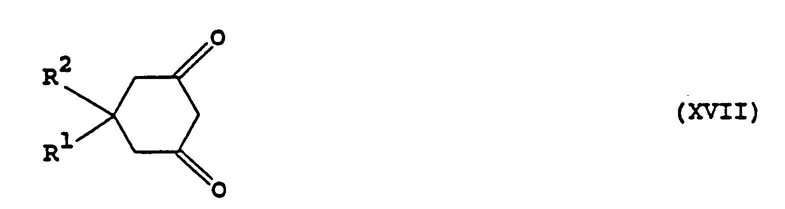 in which R¹ and R² are as defined in claim 1, with a compound of the general formula 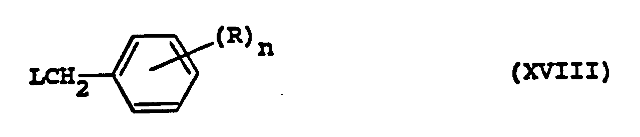 in which R and n are as defined in claim 1 and L represents a leaving group.

## Patentansprüche

1. Verfahren zur Herstellung einer Verbindung der allgemeinen Formel 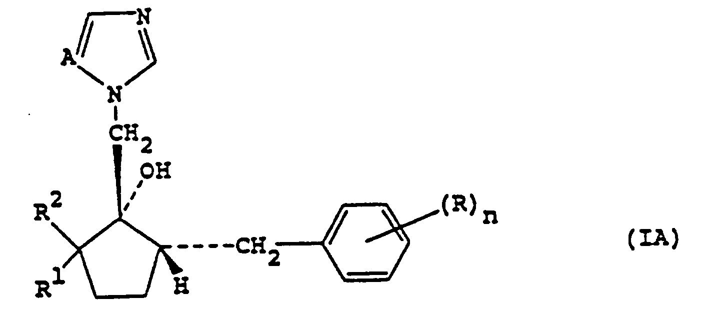 oder eines Säureadditionssalzes oder eines Metallkomplexes davon, in der n eine ganze Zahl von 0 bis 5 bedeutet, jedes R bedeutet ein Halogenatom eine Nitro-, Cyano-, Alkyl- Halogenalkyl- oder Phenylgruppe; R¹ und R² bedeuten unabhängig voneinander ein Wasserstoffatom oder eine Alkylgruppe; und A bedeutet ein Stickstoffatom oder eine CH-Gruppe, umfassend das Umsetzen einer Verbindung der allgemeinen Formel 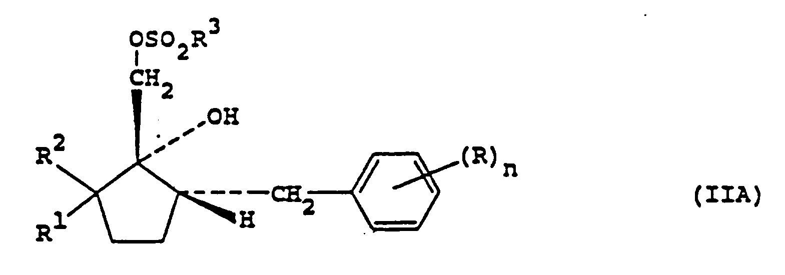 in der n, R, R¹ und R² wie oben definiert sind, und R³ eine wahlweise substituierte Alkyl- oder Arylgruppe bedeutet, mit einer Verbindung der allgemeinen Formel 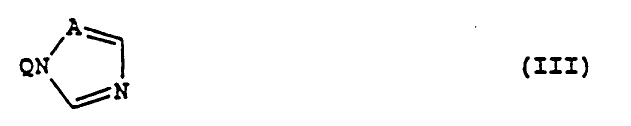 in der A wie oben definiert ist, und Q ein Wasserstoff oder ein Alkalimetallatom bedeutet, in Gegenwart einer Base; und wahlweise Umwandeln der erhaltenen Verbindung der Formel IA in sein Säureadditionssalz oder ein Metallkomplex davon.

2. Verfahren nach Anspruch 1, worin die Verbindung der Formel IIA hergestellt wird durch Umsetzen einer Verbindung der allgemeinen Formel 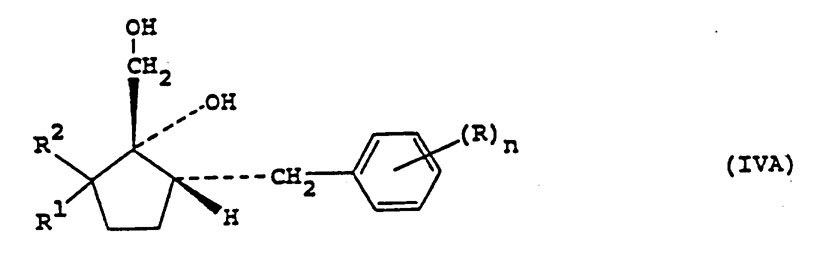 in der n, R, R¹ und R² wie in Anspruch 1 definiert sind, mit einer Verbindung der allgemeinen Formel
R³SO₂X (V)
in der R³ wie in Anspruch 1 definiert ist und X und Halogenatom bedeutet, in Gegenwart einer Base.

3. Verfahren nach Anspruch 2, worin die Verbindung der Formel IVA hergestellt wird entweder durch
(a) Umsetzen einer Verbindung der allgemeinen Formel 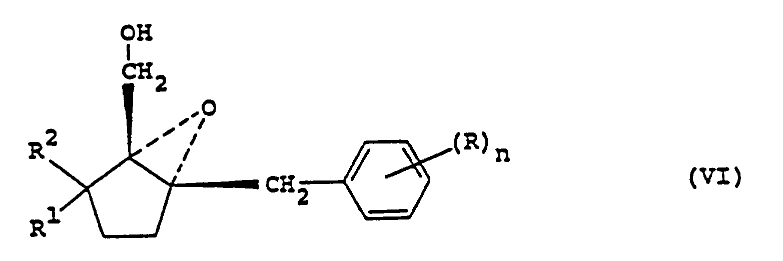 in der n, R, R¹ und R² wie in Anspruch 1 definiert sind, mit einem Reduktionsmittel bei einer Temperatur von 35°C bis Rückflußtemperatur; oder
(b) durch Umsetzen einer Verbindung der allgemeinen Formel 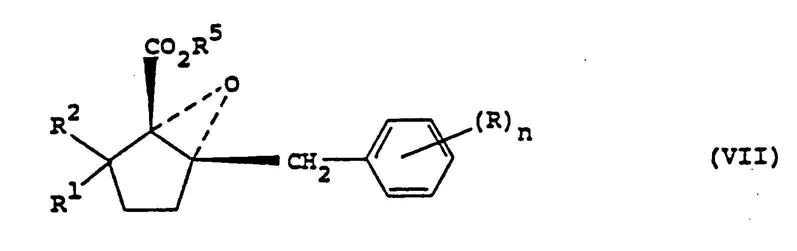 in der n, R, R¹ und R² wie in Anspruch 1 definiert sind, und R5 ein Wasserstoffatom oder eine Alkylgruppe bedeutet, mit einem Reduktionsmittel bei einer Temperatur von 35°C bis Rückflußtemperatur.

4. Verfahren nach Anspruch 3, worin die Verbindung der Formel VI hergestellt wird durch Umsetzen einer Verbindung der Formel VII, wie in Anspruch 3 definiert, mit einem Reduktionsmittel bei einer Temperatur von Raumtemperatur bis 85°C.

5. Verfahren nach Anspruch 3 oder Anspruch 4, worin die Verbindung der Formel VII hergestellt wird durch Umsetzen einer Verbindung der allgemeinen Formel 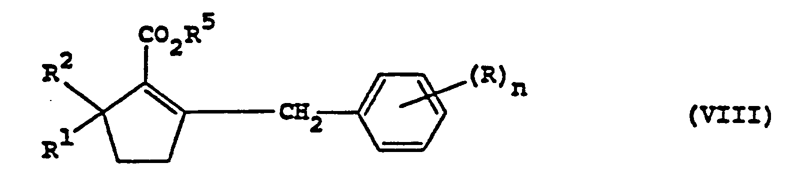 in der n, R, R¹ und R² wie in Anspruch 1 definiert sind, und R⁵ wie in Anspruch 3 definiert ist, mit einer Persäure.

6. Verfahren nach Anspruch 5, in der die Verbindung der Formel VIII hergestellt wird durch Erhitzen einer Verbindung der allgemeinen Formel 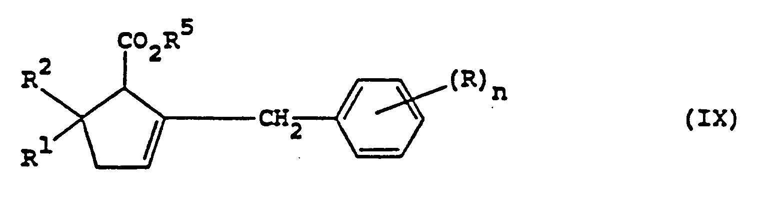 oder durch Umsetzen der allgemeinen Formel 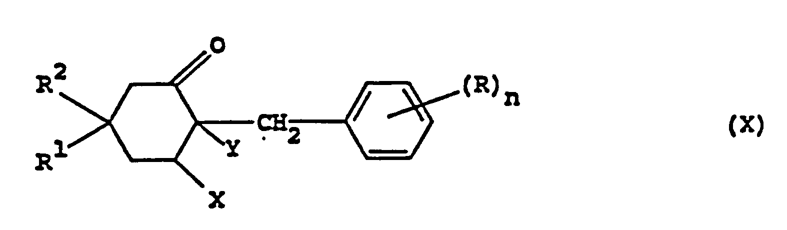 in der n, R, R¹ und R² wie in Anspruch 1 definiert sind, R⁵ ist wie in Anspruch 3 definiert, X ist wie in Anspruch 2 definiert und Y bedeutet ein Halogenatom, mit einer Verbindung der allgemeinen Formel
MOR⁵ (XI)
in der R⁵ wie in Anspruch 3 definiert ist, und M ein Alkalimetallatom bedeutet, in Gegenwart eines polaren Lösungsmittels.

7. Verfahren nach Anspruch 6, worin die Verbindung der Formel IX hergestellt wird durch Umsetzen einer Verbindung der Formel X, wie in Anspruch 6 definiert, mit einer Verbindung der Formel XI, wie in Anspruch 6 definiert, in Gegenwart eines Lösungsmittels der Formel XII
R⁵OH (XII)
worin R⁵ wie in Anspruch 3 definiert ist.

8. Verfahren nach Anspruch 6 oder 7, worin die Verbindung der Formel X hergestellt wird durch Umsetzen einer Verbindung der allgemeinen Formel 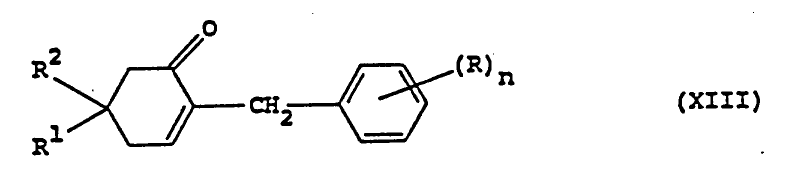 in der n, R, R¹ und R² wie in Anspruch 1 definiert sind, mit einer Verbindung XY, in der X wie in Anspruch 2 definiert ist und Y wie in Anspruch 6 definiert ist.

9. Verfahren nach Anspruch 8, in der die Verbindung der Formel XIII hergestellt wird durch Umsetzen einer Verbindung der allgemeinen Formel 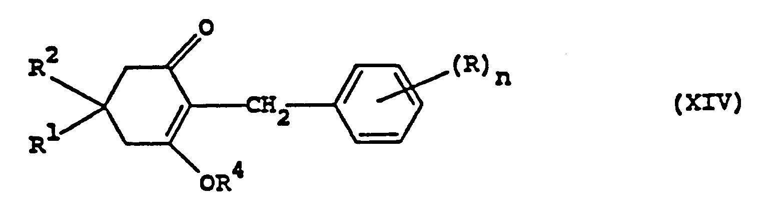 worin n, R, R¹ und R² wie in Anspruch 1 definiert sind und R⁴ eine Alkylgruppe bedeutet, mit einem Reduktionsmittel und anschließender Hydrolyse der Reaktionsmischung.

10. Verfahren nach Anspruch 9, worin die Verbindung der Formel XIV hergestellt wird durch Umsetzen einer Verbindung der allgemeinen Formel 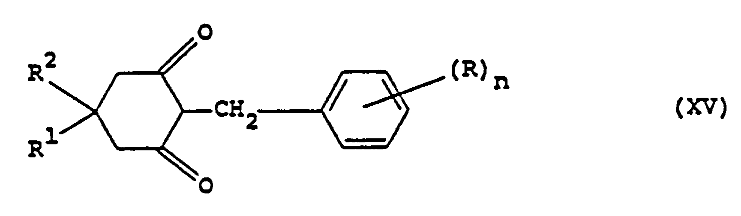 in der n, R, R¹ und R² wie in Anspruch 1 definiert sind, mit einer Verbindung der allgemeinen Formel
R⁴OH (XVI)
in der R⁴ wie in Anspruch 9 definiert ist, in Gegenwart einer Säure.

11. Verfahren nach Anspruch 10, worin die Verbindungen der Formel XV hergestellt wird durch Umsetzen einer Verbindung der allgemeinen Formel 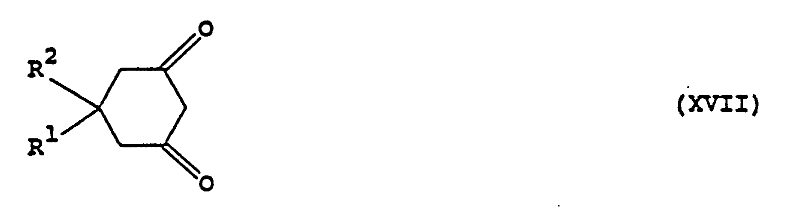 in der R¹ und R² wie in Anspruch 1 definiert sind, mit einer Verbindung der allgemeinen Formel 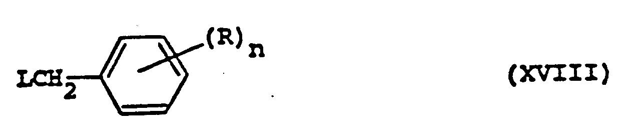 in der R und n wie in Anspruch 1 definiert sind, und L eine Austrittsgruppe ist.

## Revendications

1. Procédé de préparation d'un composé de la formule générale : 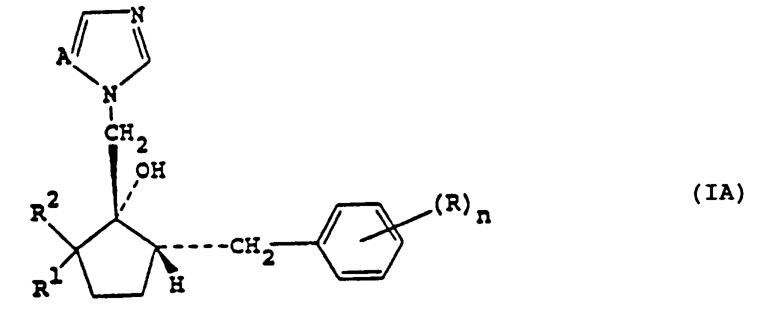 ou d'un sel d'addition d'acide ou d'un complexe métallique de celui-ci, dans lequel n représente un nombre entier de 0 à 5, chaque R représente un atome d'halogène, un groupe nitro, cyano, alkyle, haloalkyle ou phényle; R¹ et R² représentent indépendamment un atome d'hydrogène ou un groupe alkyle; et A représente un atome d'azote ou un groupe CH; qui comprend la réaction d'un composé de la formule générale : 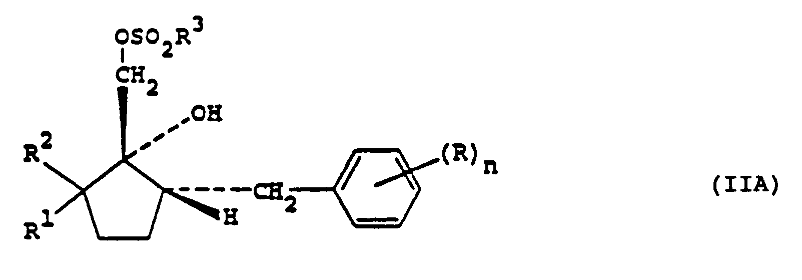 dans laquelle n, R, R¹ et R² sont comme définis ci-dessus et R³ représente un groupe alkyle ou aryle éventuellement substitué; avec un composé de la formule générale : 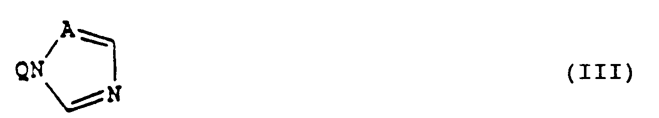 dans laquelle A est comme défini ci-dessus et Q représente un atome d'hydrogène ou de métal alcalin, en la présence d'une base; et, éventuellement, la conversion du composé obtenu de la formule Ia en un sel d'addition d'acide ou un complexe métallique de celui-ci.

2. Procédé suivant la revendication 1, dans lequel le composé de la formule IIA est préparé en faisant réagir un composé de la formule générale : 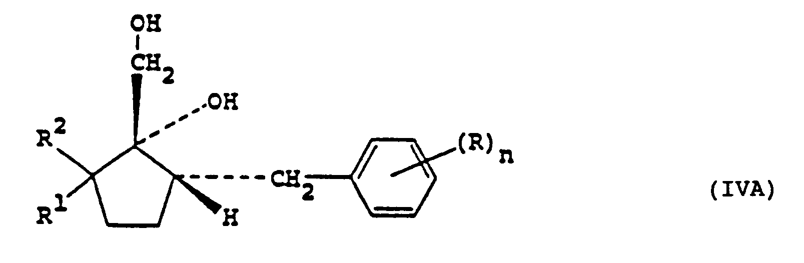 dans laquelle n, R, R¹ et R² sont comme définis dans la revendication 1, avec un composé de la formule générale :
R³SO₂X (V)
dans laquelle R³ est comme défini dans la revendication 1 et X représente un atome d'halogène, en la présence d'une base.

3. Procédé suivant la revendication 2, dans lequel le composé de la formule IVA est préparé soit
(a) en faisant réagir un composé de la formule générale: 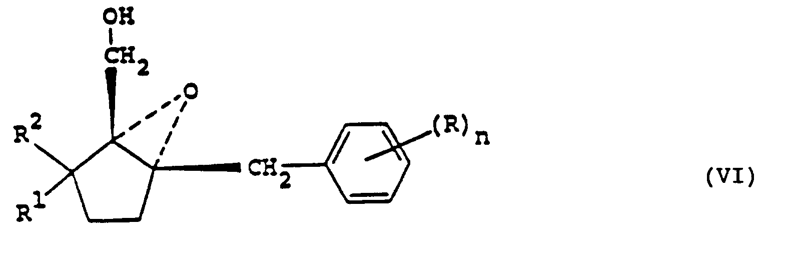 dans laquelle n, R, R¹ et R² sont comme définis dans la revendication 1, avec un agent de réduction à une température allant de 35°C à la température de reflux ; ou
(b) en faisant réagir un composé de la formule générale: 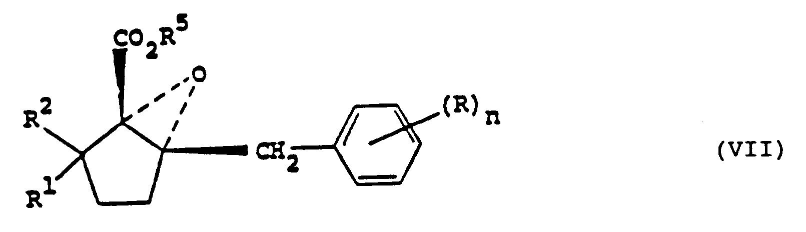 dans laquelle n, R, R¹ et R² sont comme définis dans la revendication 1, et R⁵ représente un atome d'hydrogène ou un groupe alkyle, avec un agent de réduction à une température allant de 35°C à la température de reflux.

4. Procédé suivant la revendication 3, dans lequel le composé de la formule VI est préparé en faisant réagir un composé de la formule VII, comme défini dans la revendication 3, avec un agent de réduction à une température allant de la température ambiante à 85°C.

5. Procédé suivant la revendication 3 ou la revendication 4, dans lequel le composé de la formule VII est préparé en faisant réagir un composé de la formule générale : 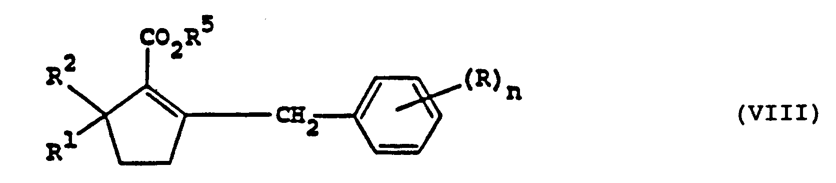 dans laquelle n, R, R¹ et R² sont comme définis dans la revendication 1, et R⁵ est comme défini dans la revendication 3, avec un peracide.

6. Procédé suivant la revendication 5, dans lequel le composé de la formule VIII est préparé en chauffant un composé de la formule générale : 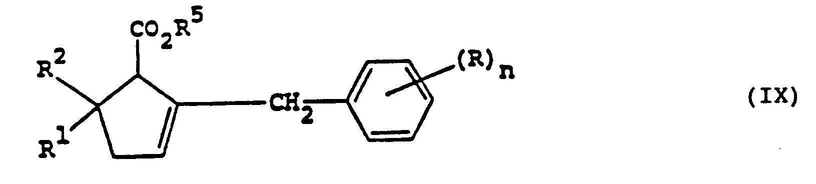 ou en faisant réagir un composé de la formule générale : 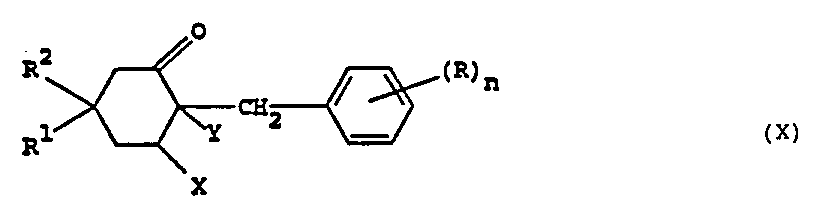 dans laquelle n, R, R¹ et R² sont comme définis dans la revendication 1, R⁵ est comme défini dans la revendication 3, X est comme défini dans la revendication 2 et Y représente un atome d'halogène, avec un composé de la formule générale :
MOR⁵ (XI)
dans laquelle R⁵ est comme défini dans la revendication 3 et M représente un atome de métal alcalin, en la présence d'un solvant polaire.

7. Procédé suivant la revendication 6, dans lequel le composé de la formule IX est préparé en faisant réagir un composé de la formule X, comme défini dans la revendication 6, avec un composé de la formule XI, comme défini dans la revendication 6, en la présence d'un solvant de la formule (XII) :
R⁵OH (XII)
dans laquelle R⁵ est comme défini dans la revendication 3.

8. Procédé suivant la revendication 6 ou la revendication 7, dans lequel le composé de la formule X est préparé en faisant réagir un composé de la formule générale : 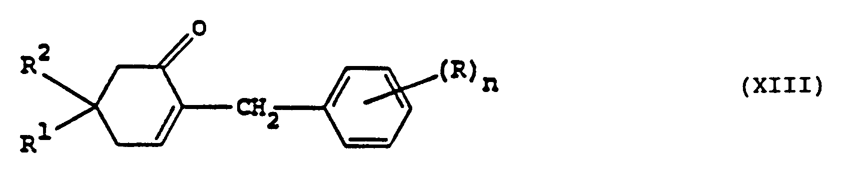 dans laquelle n, R, R¹ et R² sont comme définis dans la revendication 1, avec un composé XY dans lequel X est comme défini dans la revendication 2 et Y est comme défini dans la revendication 6.

9. Procédé suivant la revendication 8, dans lequel le composé de la formule XIII est préparé en faisant réagir un composé de la formule générale : 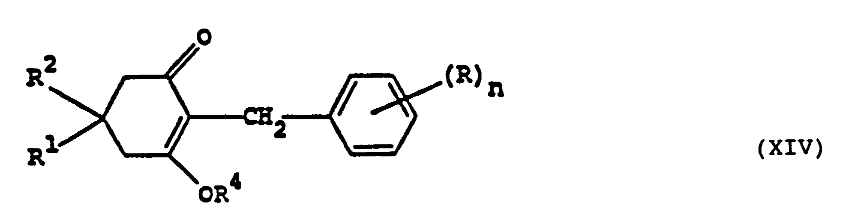 dans laquelle n, R, R¹ et R² sont comme définis dans la revendication 1 et R⁴ représente un groupe alkyle, avec un agent de réduction, et en hydrolysant ultérieurement le mélange réactionnel.

10. Procédé suivant la revendication 9, dans lequel le composé de la formule XIV est préparé en faisant réagir un composé de la formule générale : 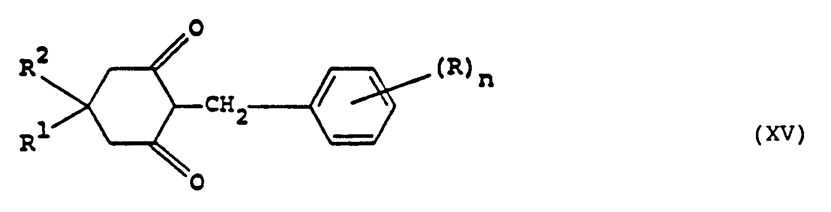 dans laquelle n, R, R¹ et R² sont comme définis dans la revendication 1, avec un composé de la formule générale :
R⁴OH (XVI)
dans laquelle R⁴ est comme défini dans la revendication 9, en la présence d'un acide.

11. Procédé suivant la revendication 10, dans lequel le composé de la formule XV est préparé en faisant réagir un composé de la formule générale : 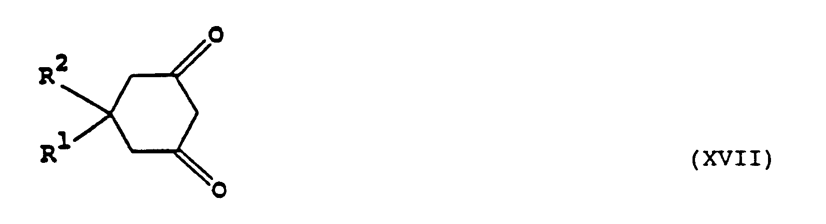 dans laquelle R¹ et R² sont comme définis dans la revendication 1, avec un composé de la formule générale : 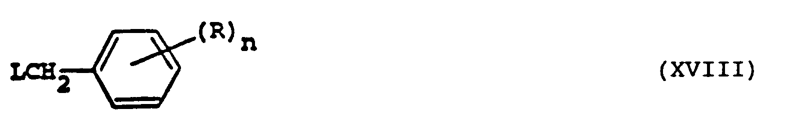 dans laquelle R et n sont comme définis dans la revendication 1 et L représente un groupe partant.
